# EUROPEAN PATENT SPECIFICATION

(11) **EP 2 032 048 B1**
(45) Date of publication and mention of the grant of the patent: **16.03.2016**
(21) Application number: 07796249.6
(22) Date of filing: 19.06.2007
(51) Int. Cl.: A61B 17/221

(54) **IMPROVED HANDLE FOR LITHOTRIPSY BASKET DEVICE**
VERBESSERTER GRIFF FÜR LITHOTRIPSIEKORBVORRICHTUNG
POIGNÉE AMÉLIORÉE POUR SONDE DE LITHOTRITIE À PANIER

(30) Priority: 26.06.2006 US 816526 P
(43) Date of publication of application: 11.03.2009
(73) Proprietor: Cook Medical Technologies LLC, Bloomington, IN 47404 (US)
(72) Inventor: KENNEDY, Kenneth, C., II, Clemmons, NC 27012 (US)
(74) Representative: Garratt, Peter Douglas
(86) International application number: PCT/US2007/014255
(87) International publication number: WO 2008/002417

(56) References cited:
- WO-A-2006/060433
- DE-A1- 3 626 371
- US-A- 4 768 505
- US-A- 5 788 710
- US-B1- 6 210 398
- US-B1- 6 217 587

## Description

### TECHNICAL FIELD

The present invention relates generally to medical devices, and relates more specifically to devices for mechanical lithotripsy of stones (calculi) such as bile stones.

### BACKGROUND

The gall bladder is an organ that stores bile secreted by the liver. The cystic duct from the gall bladder merges with the common hepatic duct, forming the common bile duct. A number of medical conditions are associated with various disorders, diseases, and injuries associated with the bile duct.

Choledocholithiasis is a medical condition associated with the entry of a biliary calculus (bile stone) into the bile duct. Obstruction of the bile duct can be excruciatingly painful for a patient suffering therefrom, and can cause nausea, fever, vomiting, and jaundice. Complete, persistent obstruction of the common bile duct can cause cholangitis, a life threatening infection of the biliary tree, which is a medical emergency. An obstruction of the common bile duct can also lead to an obstruction of the pancreatic duct, which may cause pancreatitis.

Several methods of treatment are used to remove the gall bladder and stones, including open surgery or laparoscopic surgery. Less invasive treatments may be used as well. For example, the stones may be removed endoscopically, without having to create any external incisions. In this technique, an endoscope is directed through the patient's esophagus to a location adjacent the Sphincter of Oddi, where the bile duct opens into the duodenum. Typically, a sphincterotome is used to cannulate and widen the sphincter opening to ease access into the bile duct for stone retrieval. A device including a basket deployable from a lumen of a catheter may then be directed into the bile duct to capture stones for removal.

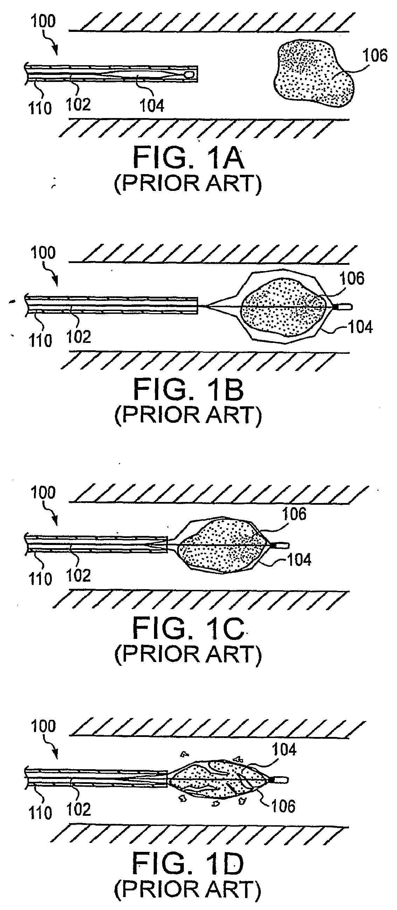

In some instances the stones are too large to pass through even the widened Sphincter of Oddi. If more invasive surgical techniques are to be avoided, then the stone must be crushed or broken into smaller pieces for removal (lithotripsy). A number of devices are known in the art for breaking up the stones. One such device is a mechanical lithotriptor basket device 100 comprising a wire basket 104 mounted on the distal end of an elongate basket wire 102, which is guided through a catheter 110 to a location such that the basket 104 can be directed around a stone 106 (See FIGS. 1A-1C). Once the basket 104 is around the stone 106, the basket 104 is retracted toward and into the catheter 110, such that its internal volume is reduced. The compressive force caused thereby breaks or crushes the stone 106 into smaller pieces (See FIG. 1 D) so that it can be removed or allowed to pass.

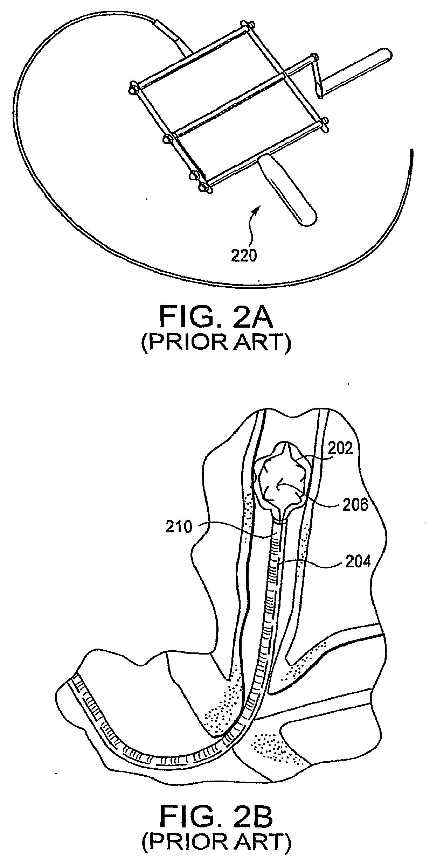

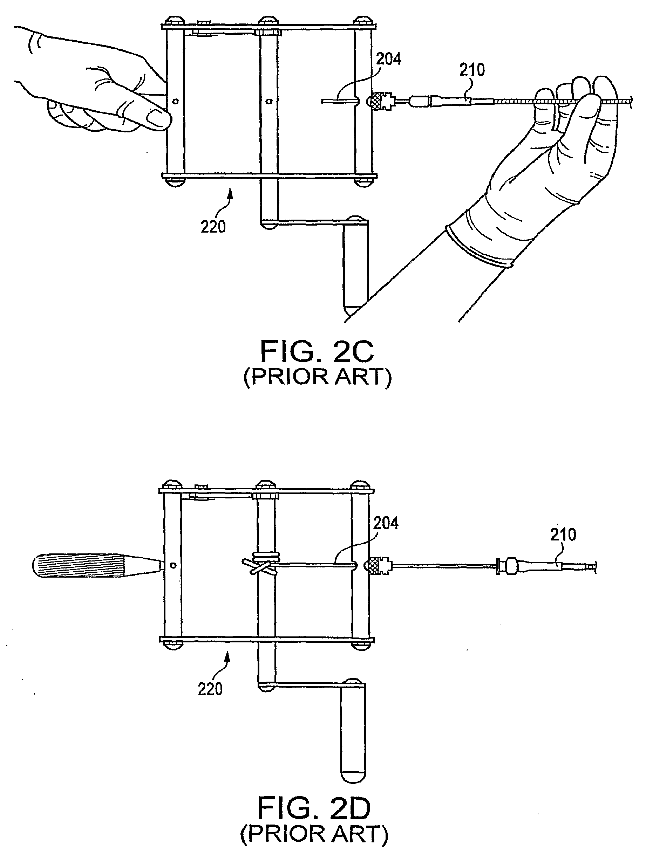

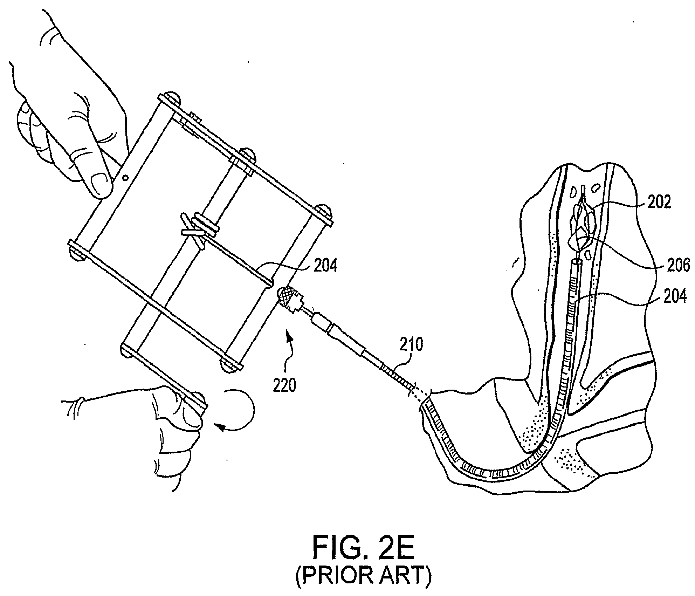

In some circumstances, the retraction and compaction of the basket 104 may be accomplished by a user directly pulling the basket wire 102 proximally (e.g., with a standard handle such as a three-ring handle or a flanged-spool/stem handle). However, because some stones may be resistant, it is often necessary to provide mechanical advantage to aid in crushing of the stone 106. One device that has been used for this purpose is a reel-type device embodied in the Soehendra® Mechanical Lithotriptor (Cook Endoscopy). FIG. 2A illustrates a reel-type lithotriptor accessory handle 220 and FIGS. 2B-2E depict a method of use. FIG. 2B shows the distal portion of a lithotripsy device 200 including a lithotripsy basket 202 at the distal end of a basket wire 204 and catheter 210 fully engaged with a stone 206. FIGS. 2C-2D depict how the proximal end of the basket wire 204 and catheter 210 are mounted to the lithotriptor accessory handle 220 after removal of an initial proximal structure (such as, for example, a three-ring handle). FIG. 2E shows how the lithotriptor accessory handle 220 is actuated to crush the stone 206. Other presently-available devices for providing mechanical advantage when a stone is resistant to crushing also require the use of additional accessory tools that must be assembled to the lithotripsy device 200 to provide mechanical advantage. This requirement of extra steps and extra hardware reduce the efficiency of time and effort that is most desirable during surgical procedures. Thus, there is a need for a lithotripsy device that provides a standard-use handle equipped to provide added mechanical advantage without requiring other devices and/or time-consuming adaptation of a basic lithotriptor during prosecution. Reference is directed to US 5,788,710, which is the prior art upon which the two-part form of Claim 1 is based, and which relates to lithotripsy and discloses an instrument having a handle, a catheter and a basket. An operating assembly in the handle includes a manual control knob directly connected to the basket and a trigger coupled to the basket to retrieve the basket with mechanical advantage. A ratchet switch permits selective engagement and disengagement of a ratchet stop. Reference is also directed to US 4,768,505 which discloses calculus crushing apparatus including a flexible sheath for insertion into a body cavity and an operation wire with a basket fixed to the distal end. By moving the connecting rod by an operation mechanism, the basket is projected from or retracted in the sheath.

### BRIEF SUMMARY

The scope of the present invention is set forth in the appended claims. Embodiments of the present disclosure will provide improved mechanical advantage without need for assembly of other structures to the handle. In one aspect, there is disclosed a lithotriptor device that has a proximal handle, an elongate sheath with a lumen extending therethrough; and a basket distally attached to a basket wire, the wire extending through the lumen of the elongate sheath. The handle includes a first handle member and a second handle member that is axially movable relative to the first handle member. The first handle member is connected to the elongate sheath, and the second handle member is connected to the basket wire. The first and second handle members each comprising an engagement member that is configured to be engaged by a hand of the user. The second handle member also includes at least one lever and pawl assembly wherein the pawl is rotatably attached near a first end of the lever and is biased in engagement with a distal portion of the first handle member, and wherein a fulcrum of the lever connects the lever to the second handle member. The handle has a first mode of operation comprising a direct axial sliding movement of the first handle member along the second handle member, and a second mode of operation providing mechanical advantage. The second mode of operation includes providing a generally proximal movement of a second end of the lever, which forces the pawl against the distal portion of the first handle member with sufficient force to move the connected elongate sheath distally relative to the wire.

In another aspect, there is disclosed a lithotriptor device having a proximal end and a distal end, and including an elongate shaft with a lumen extending therethrough. A wire extends through the lumen of the elongate shaft. A first handle member has an attachment to the shaft, and a second handle member is mounted to the first handle member in a manner that allows a generally axial movement of the second handle member relative to the first handle member. The second handle member includes an attachment to the wire such that when the second handle member is moved in a proximal direction relative to the first handle member, the wire is pulled in the proximal direction relative to the lumen of the shaft. The second handle member also includes at least one lever configured to provide mechanical advantage for moving the second handle member proximally relative to the first handle member.

In yet another aspect, there is disclosed a lithotriptor device having a proximal end and a distal end. The lithotriptor includes a first handle member connected to an elongate shaft. The elongate shaft has a lumen extending therethrough. A second handle member is mounted to the first handle member in a manner allowing movement of the first handle member relative to the second handle member. The second handle member includes a connection to a basket wire that is disposed through the lumen of the elongate shaft. The second handle member also includes a means for providing mechanical advantage when moving the first handle member relative to the second handle member. The lithotriptor is configured such that a movement of the first handle member relative to the second handle member moves the basket wire relative to the elongate shaft.

In still another aspect, there is disclosed a method for disrupting the integrity of an object (such as, for example, by breaking or crushing it). The method includes several steps: One step is providing a lithotriptor device, which has a proximal handle, an elongate sheath with a lumen extending therethrough; and a basket distally attached to a basket wire the wire extending through the lumen of the elongate sheath. The lithotriptor handle has a first handle member and a second handle member that is axially movable relative to the first handle member. The first handle member is connected to the elongate sheath, and the second handle member is connected to the basket wire. The second handle member also includes at least one lever and pawl, wherein the pawl is rotatably attached near a first end of the lever and is biased in engagement with a region of the first handle member, and wherein a fulcrum of the lever connects the lever to the second handle member. Another step is engaging the basket around an object. Yet another step is moving the second handle member relative to the first handle member such that the basket wire is drawn proximally into the elongate sheath and the basket is drawn tightly around the object. Yet another step is actuating the at least one lever and pawl assembly by operating the lever such that the pawl's engagement with the first handle member moves the elongate sheath distally relative to the basket wire.

### BRIEF DESCRIPTION OF THE DRAWINGS

FIGS. 1A-1D depict the function of a lithotriptor basket;
FIG. 2A illustrates a prior art lithotriptor handle accessory for increasing mechanical advantage;
FIG. 2B shows a lithotriptor basket engaging a biliary calculus;
FIGS. 2C-2E depict a method of using the prior art lithotriptor handle accessory with a lithotripsy device;
FIGS. 3A-3D illustrate a first lithotriptor device and methods of use;
FIGS. 4A-4C show a second lithotriptor device , which illustrates the invention, and methods of use;
FIGS. 5A-5B depict a third lithotriptor device; and
FIG. 5C illustrates a cross-sectional view of a handle member of FIG. 5B.

### DETAILED DESCRIPTION

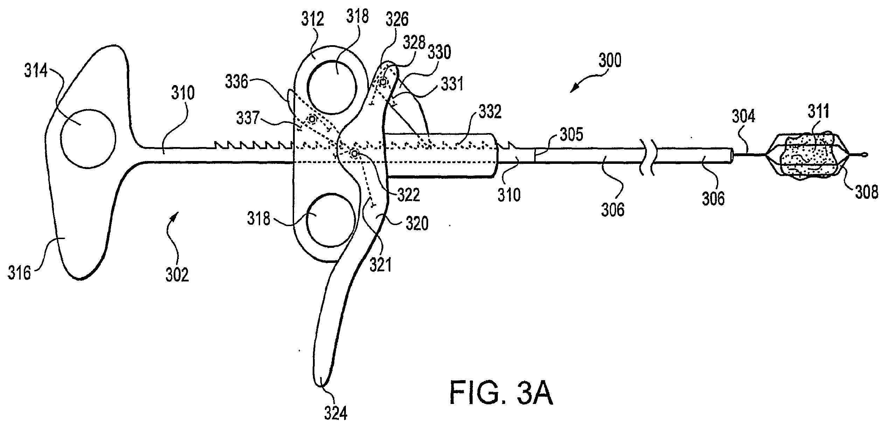

A first example of a handle 302 for a lithotriptor 300 is illustrated in FIGS 3A-3D. In addition to the handle 302, the lithotriptor 300 includes a basket wire 304, circumscribed by and axially slidable within an outer sheath 306. The distal end of the basket wire 304 includes a lithotripsy basket 308, which is shown in FIG. 3A as engaging a biliary stone 311. The handle 302 includes a modified three-ring handle design. The stem (thumb-ring) portion 310 is attached to the proximal end 305 of the outer sheath 306. The spool (finger-ring) portion 312 is attached to the basket wire 304 such that axial movement of the spool 312 relative to the stem 310 causes corresponding axial movement of the basket wire 304 within the outer sheath 306. In preferred examples, the handle will be constructed of materials known in the art to be durable and suited for multiple sterilizations such as metals, resins, composites, or combinations thereof. For a disposable handle, certain injection-molded polymers may be appropriate. In preferred examples, load-bearing pivot points/axes (e.g., pivot pins) will be made of steel or a similarly rigid and durable material.

The proximal portion of the stem 310 includes a thumb ring aperture 314. A broad body 316 surrounding the aperture 314 preferably is shaped to fit comfortably in a user's palm during an operation when the spool 312 is pulled along the stem 310 toward the proximal end. The spool 312 includes two finger ring apertures 318. Thus, the handle 302 includes structure that allows a user comfortably to move the spool 312 distally along the stem 310 by engaging her fingers into the finger ring apertures 318 and either engaging her thumb into the thumb ring aperture 314 or placing the broad proximal body 316 against her palm.

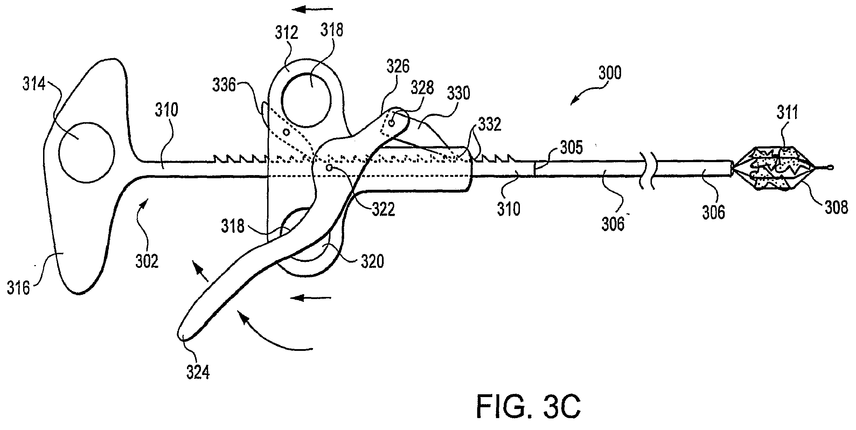

The handle 302 also includes a ratcheting lever mechanism to provide mechanical advantage during a stone-crushing operation. The spool 312 includes a lever 320 attached at a first pivot axis (fulcrum) 322. The pivot axis 322 is shown as being generally centered along the breadth of the spool 312 and aligned with the central longitudinal axis of the stem 310, but it may be located more toward one or the other of the finger ring apertures 318 in other embodiments. The lever 320 is pivotable within a plane defined by the longitudinal axes of the stem 310 and the spool 312. The longer, effort end 324 of the lever 320 extends beyond the lower side of the spool 312 and includes an aperture that allows the lever to be moved at least partially over the lower side of the spool 312 as shown in FIG. 3C. The effort end 324 of the lever optionally may include an open or closed loop structure for gripping (e.g., like a scissors handle). The shorter, load end 326 of the lever 320 includes a second pivot axis 328 connecting it to the proximal end of a driver pawl 330. The distal end of the pawl 330 engages ratchet teeth 332 on a distal surface of the stem 310. Preferably, a torsion spring 331 or other spring-type structure biases the pawl 330 into engagement with the ratchet teeth. Also preferably, a torsion spring 321 or other spring-type structure (not shown) biases the lever 320 into a first position shown in FIG. 3A. (These may be, for example, the type of torsion spring available from Master Spring and Wire Form Co., River Grove, IL.)

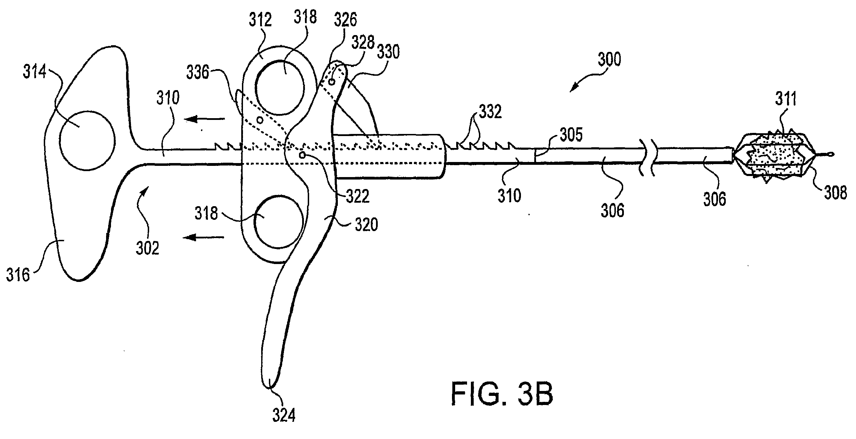

As is illustrated with reference to FIG. 3B, the handle 302 may be actuated in the same fashion as a standard three-ring handle by pulling the spool 312 proximally along the stem 310 and toward the broad proximal body 316 using the finger ring apertures 318. However, the handle 302 of the present embodiment also provides the ability to apply additional force (mechanical advantage) when needed to crush or otherwise disrupt the physical integrity of a recalcitrant stone 311, without the need for attachment of additional tools.

To provide mechanical advantage in retracting the basket wire 304 such that the basket 308 may exert crushing compressive force on the stone 311 as it is drawn closed by the outer shaft 306, a user may actuate the lever 320. Actuation of the lever 320 in a compression stroke includes pulling the effort end 324 proximally as shown in FIG. 3C. This action levers the driver pawl 330 distally against the ratchet teeth 332 on the stem 310, forcing the outer sheath 306 in a distal direction (relative to the spool 312, or retracting the basket wire proximally into the sheath - those of skill in the art appreciating that the actuating movement is relative movement). The resulting distal movement of the outer sheath 306 (relative to the basket wire 304) compresses the basket 308 to crush the stone 311.

In a preferred example, a retaining pawl 336 is mounted to the spool 312 and is releasably biased into engagement with ratchet teeth 332 that are proximal of the driver pawl 330 by, for example, a torsion spring 337 attached to the retaining pawl 336 and the spool. This helps to prevent the outer sheath 306 from creeping back proximally (e.g., as the stone 311 and/or basket resist compression). Specifically, the retaining pawl 336 maintains an engagement with the ratchet teeth 332 such that the driver pawl 330 may be disengaged from the ratchet teeth 332. With the retaining pawl 336 engaged, even if there is distalward tension on the basket wire 304 relative to the spool 312, the engagement of the retaining pawl 336 with the ratchet teeth 332 on the stem 310 prevents the spool and basket wire from moving distally in a manner that would relieve the pressure being exerted on the stone 311 when a user disengages the driver pawl 330 to move the lever 320 back to its original position (relative to the spool 312) for another actuation stroke. Those of skill in the art will appreciate that more than one set of ratchet teeth may be provided such that a driver pawl and a retaining pawl engage different sets of ratchet teeth. In addition, it will also be appreciated that one or both ratchet/pawl mechanisms of this and other embodiments may be replaced with a frictional retention means such as, for example a pawl having a frictional surface configured to engage with a surface on one of the handle members.

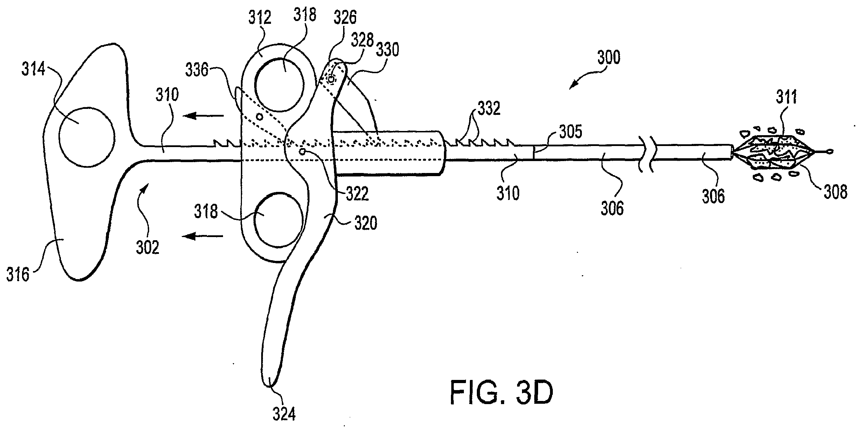

The torsion spring 331, which biases the driver pawl 330 engagingly with the ratchet teeth 332, allows the lever 320 to be returned to its initial position during a return stroke (as shown in FIG. 3D) while maintaining contact between the driver pawl 330 and the ratchet teeth 332. As the lever 320 is returned to its initial position (relative to the spool 312), the driver pawl 330 "clicks over" the ratchet tooth (or teeth) 332 proximal of its extended/actuated position. (NOTE: the torsion springs 321, 331, 337 are shown only in FIG. 3A; the tines of springs 331 and 337 are biased toward each other, respectively, and the tines of spring 321 are biased away from each other). In some embodiments, each of the pawls 330 and 336 may be configured to be releasable (e.g., by an over-center mounting or other means known to those of skill in the art) in a manner allowing distal movement of the spool 312 relative to the stem 310.

Thus, if desired, a user may actuate the lever 320 several times to advance the outer sheath 306 for compressing the basket 308, after which the outer sheath 306 will have been advanced sufficiently to provide crushing force on the stone as shown in FIG. 3D. The length of each stroke with the lever 320 may be selected by the user to advance the outer sheath 306 by a desired amount that may correspond to one or more of the ratchet teeth 332. It should be noted that the retaining pawl 336 or another gripping retaining member could be located distally of the driver pawl, or in another position. In alternative examples (not shown), the engagement of the pawls 330, 336 with the stem 310 or the outer sheath 306 need not include ratchet teeth. For example, the surface of one or both pawls, the stem, and/or the sheath may be configured to engage frictionally.

In a preferred method of use, the user of the lithotriptor 300 first engages the basket 308 around the stone 311 and uses the handle 302 in a standard three-ring fashion to draw the basket 308 snugly around the stone 311 to crush it (see FIG. 3B). If the force available by "thumb and fingers" actuation of the handle 302 in "traditional three-ring handle mode" is insufficient to crush the stone, the.user then disengages his thumb from the thumb aperture 314 and braces the broad body 316 against his palm. The user also disengages his fingers from the finger apertures 318 in the spool 312 and uses them to actuate the lever one or more times in a compression stroke as described above with reference to FIGS. 3C-3D. The mechanical advantage provided by the lever 320 moves the outer sheath 306 distally with greater force to compact the basket 308 and crush the stone 311 (see FIGS. 3C-3D). As described, the illustrated lithotriptor embodiment 300 provides for a first, direct mode of actuation and a second, assisted mode of actuation (i.e., providing mechanical advantage) without a need for providing or assembling additional tools. Of course, it should be noted that, for a small and/or weak stone 311, a user may elect to use only the first mode if it is sufficient to crush the stone. Likewise, it should also be noted that a user may elect to forgo the first mode of actuation and utilize only the second mode of actuation to crush the stone 311.

In an alternative configuration of the first lithotriptor 300 (not shown), connections of the moving parts may be reversed such that the stem 310 may be attached to the basket wire 304 and the spool 312 may be attached to the outer sheath 306. In this configuration, actuation of the lithotriptor handle 302 comprises moving the spool 312 and stem 310 in opposing directions (i.e., the spool is moved distally and/or the stem is moved proximally relative to each other). In such an embodiment, the lever 320 may be mounted on the proximal side of the spool 312 such that pushing the lever actuates the device for advancing the outer sheath 306 distally relative to the basket wire. This configuration is less preferred than the configuration and method described above with reference to FIGS. 3A-3D, for at least the reason that a single-handed actuation moving the stem and spool apart will typically provide for a weaker mechanical force and/or less convenient means of providing force than compressing them together (e.g., when a user is manipulating the stem and spool with thumb and fingers, respectively).

Those of skill in the art will appreciate that, in another examples of a lithotriptor device, a lever may be provided without an actual driver pawl, and with a fulcrum/ pivot point mounted lower on the spool. In such a lithotriptor embodiment, the load end of the lever will function as a pawl and directly engage the stem (in a manner similar to a typical caulking gun configuration), providing mechanical advantage, but a shorter stroke than other examples described herein.

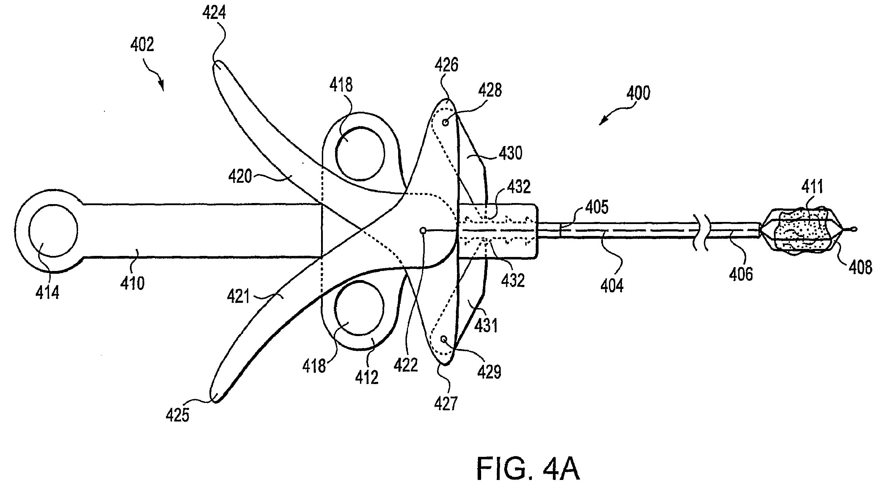

An embodiment of a handle 402 for a lithotriptor 400 in accordance with present invention is illustrated in FIGS. 4A-4C. In addition to the handle 402, the lithotriptor 400 includes a basket wire 404, circumscribed by and axially slidable within an outer sheath 406. The distal end of the basket wire 404 includes a lithotripsy basket 408, which is shown in FIG. 4A as engaging a biliary stone 411. The handle 402 includes a modified three-ring handle design. The stem (thumb-ring) portion 410 is attached to the proximal end 405 of the outer sheath 406. The spool (finger-ring) portion 412 is attached to the basket wire 404 such that axial movement of the spool 412 relative to the stem 410 causes corresponding axial movement of the basket wire 404 within the outer sheath 406.

The proximal portion of the stem 410 includes a thumb ring aperture 414. The spool 412 includes two finger ring apertures 418. Thus, the handle 402 includes structure that allows a user to move the spool 412 along the stem portion 410 by engaging her fingers into the finger ring apertures 418 and engaging her thumb into the thumb ring aperture 414, in the standard manner for operating a three-ring handle.

The handle 402 also includes a ratcheting dual lever mechanism to provide mechanical advantage during a stone-crushing operation. The spool 412 includes an opposed pair of levers 420, 421 preferably attached at a common first pivot axis (fulcrum) 422. The pivot axis 422 is shown as being generally centered and aligned with the central longitudinal axis of the stem 410, but it may be located more toward one or the other of the finger ring apertures 418 in other embodiments. The levers 420, 421 are pivotable within a plane defined by the longitudinal axes of the stem 410 and the spool 412. The longer, effort end 424, 425 of each of the levers 420, 421 extends generally proximally at an angle to the longitudinal axis of the stem 410. The effort end 424, 425 of one or both levers 420, 421 optionally may include an open or closed loop structure for gripping (e.g., like a scissors handle). The shorter, load end 426, 427 of each lever 420, 421 includes a second pivot axis 428, 429 connecting it to the proximal end of a driver pawl 430, 431. The distal end of the pawls 430, 431 engage ratchet teeth 432 on a distal surface portion of the stem 410. Preferably a torsion spring or other spring-type structure (not shown) biases each pawl 430, 431 into engagement with the ratchet teeth 432. Also preferably, a torsion spring or other spring-type structure (not shown) biases each lever 420, 421 into a first position as shown in FIG. 4A. (Note: those of skill in the art will appreciate that the paired reference numbers refer, respectively, associated components labeled with even numbers and other associated components labeled with odd numbers.)

The handle 402 may be actuated in the same fashion as a standard three-ring handle by pulling the spool 412 proximally along the stem 410 toward the thumb ring 414 using the finger ring apertures 418. However, the present handle embodiment 400 also provides the ability to apply additional force (i.e., mechanical advantage) when needed to crush a recalcitrant stone 411, without a need for attaching additional tools.

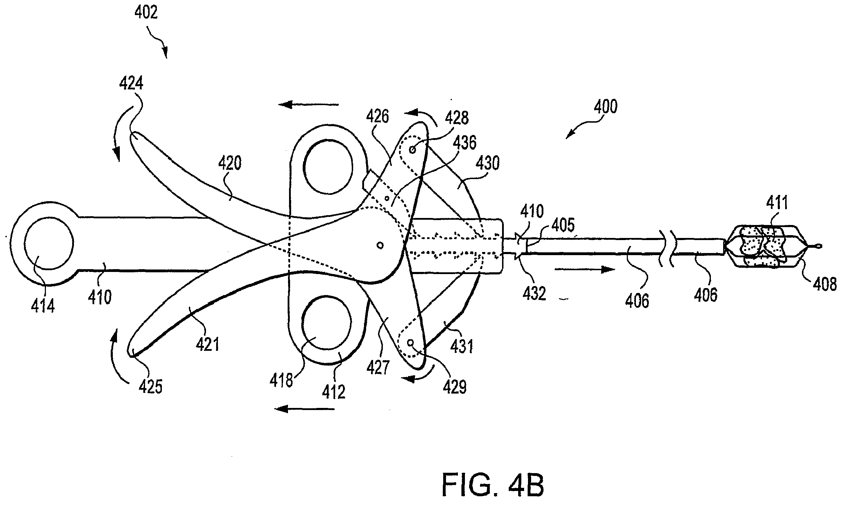

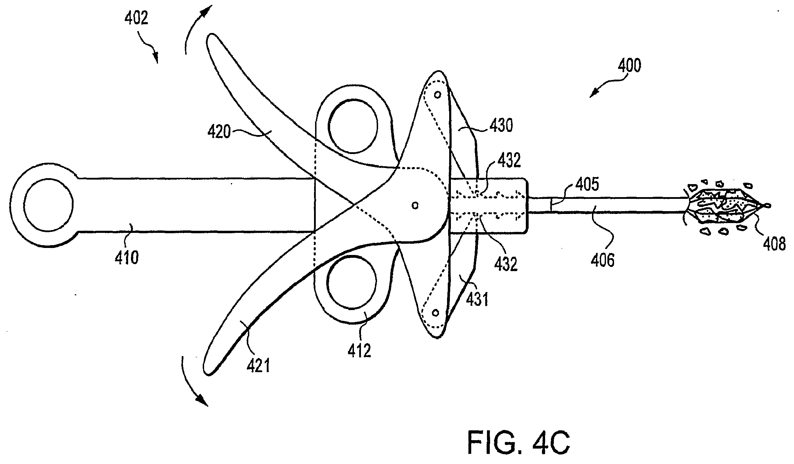

To provide mechanical advantage in retracting the basket wire 404 such that the basket 408 may exert crushing compressive force on the stone 411 as it is drawn closed by the outer shaft 406, a user actuates the levers 420, 421. Actuation of the levers 420, 421 in a compression stroke includes pressing the effort ends 424, 425 toward each other as shown in FIG. 4B. This action levers the driver pawls 430, 431 distally against the ratchet teeth 432 on the stem 410, forcing the outer sheath 406 in a distal direction relative to the basket wire 404. The resulting distal movement of the outer sheath 406 over the basket 408 compresses the basket to crush the stone 411. In a preferred embodiment, one or more retaining pawl(s) 436 are mounted to the spool 412 and biased into engagement with the ratchet teeth 432 to prevent the outer sheath 406 from creeping back proximally (e.g., as the stone 411 and/or basket resist compression). Additionally, as described above, and as depicted in FIG. 4C, the retaining pawl 436 (not shown in FIG. 4C) maintains the position of the outer sheath 406 relative to the stem 410 while the driver pawls can advance proximally to engage more-proximal ratchet teeth 432.

The torsion springs (not shown) that bias the driver pawls 430, 431 engagingly with the ratchet teeth 432 allow the levers 420, 421 to be returned to the initial "resting position" during a return stroke (see FIG. 4C) while maintaining contact between the driver pawls 430, 431 and the ratchet teeth 432. (For example, as the levers 420, 421 return to their initial "resting position," the driver pawls 430, 431 may move proximally over one or more of the ratchet teeth 432 until they can engage a more proximal set of ratchet teeth 432. See FIG. 4C). Thus, if necessary, a user may actuate the levers 420, 421 several times to advance more distally the outer sheath 406 for compressing the basket 408, after which the outer sheath 406 preferably will have been advanced sufficiently distally over the basket 408 to provide crushing force on the stone 411.

In a preferred method of use, a user of the lithotriptor 400 first engages the basket 408 around the stone 411 and uses the handle 402 in a standard three-ring fashion to draw the basket 408 snugly around the stone 411. If the force available by "thumb and fingers" actuation of the handle 402 in "traditional three-ring handle mode" is insufficient to crush the stone, the user then disengages his thumb from the thumb aperture 414 and disengages his fingers from the finger apertures 418 in the spool 412. The user then actuates the levers 420, 421 by pressing them toward each other in scissors-like fashion as described above with reference to FIGS. 4A-4C. The mechanical advantage provided by the lever 420 moves the outer sheath 406 distally with greater force to compact the basket 408 and crush the stone 411.

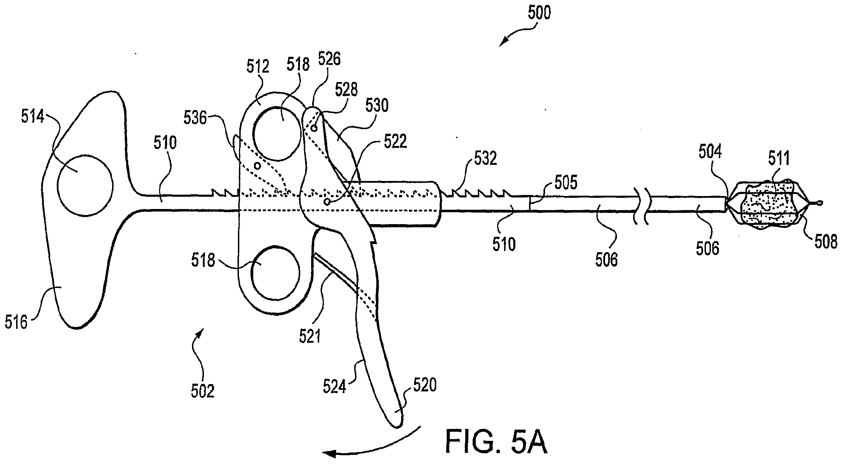

In a third example of a handle 502 for a lithotriptor 500, shown in FIGS. 5A-5C, a modified three-ring handle 502 is configured to include a lever 520 to provide mechanical advantage. This example is similar to the example shown in FIGS. 3A-3C, except that the lever providing mechanical advantage in this embodiment is positioned to have a more forward range of motion. Specifically, the effort end of the lever 520 typically will not rotate more distally than a central longitudinal axis of the spool 512. In addition to the handle 502, the lithotriptor 500 includes a basket wire 504, circumscribed by and axially slidable within an outer sheath 506. The distal end of the basket wire 504 includes a lithotripsy basket 508, which is shown in FIG. 5A as engaging a biliary stone 511. The handle 502 includes a modified three-ring handle design. The stem (thumb-ring) portion 510 is attached to the proximal end 505 of the outer sheath 506. The spool (finger-ring) portion 512 is attached to the basket wire 504 such that axial movement of the spool 512 relative to the stem 510 causes corresponding axial movement of the basket wire 504 within the outer sheath 506.

The proximal portion of the stem 510 includes a thumb ring aperture 514. A broad body 516 surrounding the aperture 514 preferably is shaped to fit comfortably in a user's palm during an operation when the spool 512 is pulled along the stem 510 toward the proximal end. The spool 512 includes two finger ring apertures 518. Thus, the handle 502 includes structure that allows a user comfortably to draw the spool 512 proximally along the stem 510 by engaging her fingers into the finger ring apertures 518 and either engaging her thumb into the thumb ring aperture 514 or placing the broad proximal body 516 against her palm.

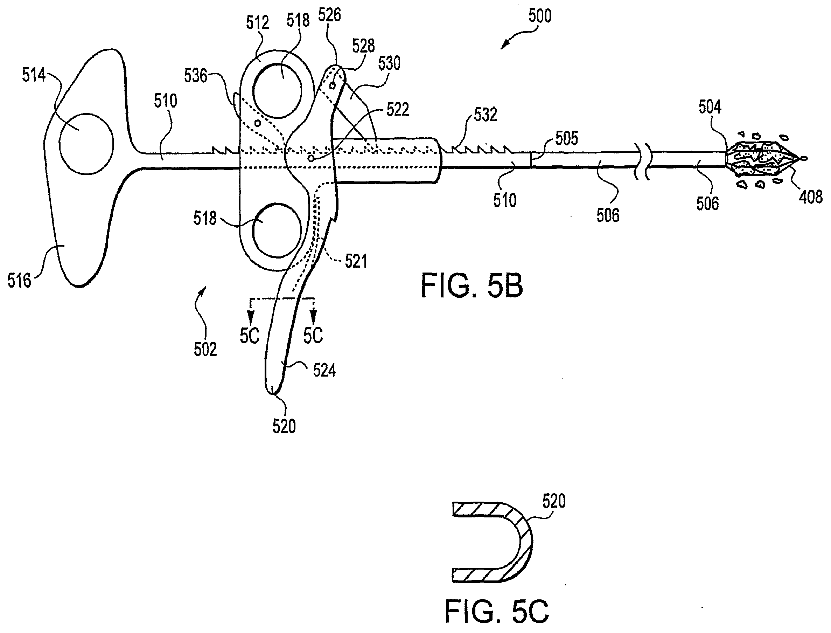

The handle 502 also includes a ratcheting lever mechanism to provide mechanical advantage during a stone-crushing operation. The spool 512 includes a lever 520 attached at a first pivot axis (fulcrum) 522. The location of the pivot axis 522 is shown as being generally transverse to and intersecting the central longitudinal axis of the stem 510, but it may be located more toward one or the other of the finger ring apertures 518 in other embodiments. The lever 520 is pivotable within a plane defined by the longitudinal axes of the stem 510 and the spool 512. When the handle 502 is in an initial resting position, the longer, effort end 524 of the lever 520 is biased such that it is angled distally away from the lower side of the spool 512. A distal contour of the lever 520 is generally U-shaped (see FIG. 5C, which is a cross-section of the handle 520 along line 5C-5C of FIG. 5B), which allows the lever to be moved at least partially over the lower side of the spool 512 as shown in FIG. 5B. The effort end 524 of the lever 520 optionally may include an open or closed loop structure for gripping (e.g., like a scissors handle). The shorter, load end 526 of the lever 520 includes a second pivot axis 528 connecting it to the proximal end of a driver pawl 530. The distal end of the pawl 530 engages ratchet teeth 532 on a distal surface of the stem 510. Preferably a torsion spring (not shown) or other biasing means biases the driver pawl 530 into engagement with the ratchet teeth. Preferably, a plastic cantilever spring 521 (or other biasing means known to those of skill in the art) biases the lever 520 into the initial resting position shown in FIG. 5A.

As will be appreciated by those of skill in the art, the handle 502 may be actuated in the same fashion as a standard three-ring handle by pulling the spool 512 proximally along the stem 510 and toward the broad proximal body 516 using the finger ring apertures 518. However, the handle 502 of the present examples also provides the ability to apply additional force (mechanical advantage) when needed to crush a recalcitrant stone 511, without the need for attachment of additional tools.

To provide mechanical advantage in retracting the basket wire 504 such that the basket 508 may exert crushing compressive force on the stone 511 as it is drawn closed by the outer shaft 506, a user actuates the lever 520. Actuation of the lever 520 in a compression stroke includes pulling the effort end 524 proximally to the orientation shown in FIG. 5B. This action levers the driver pawl 530 distally against the ratchet teeth 532 on the stem 510, forcing the outer sheath 506 in a distal direction (relative to the spool 512). The resulting distal movement of the outer sheath 506 (relative to the basket wire 504) compresses the basket 508 to crush the stone 511. In a preferred example, a retaining pawl 536 is mounted to the spool 512 and is releasably biased into engagement with ratchet teeth 532 that are proximal of the driver pawl 530 (by, for example, a torsion spring (not shown) or other biasing means known to those of skill in the art). This helps to prevent the outer sheath 506 from creeping back proximally (e.g., as the stone 511 and/or basket resist compression). Specifically, the retaining pawl 536 maintains an engagement with the ratchet teeth 532 such that the driver pawl 530 may be disengaged from the ratchet teeth 532. With the retaining pawl 536 engaged, even if there is distalward tension on the basket wire 504 relative to the spool 512, the engagement of the retaining pawl 536 with the ratchet teeth 532 on the stem 510 prevents the spool 512 and basket wire 504 from moving distally in a manner that would relieve the pressure being exerted on the stone 511 when a user disengages the driver pawl 530 to move the lever 520 back to its original position (relative to the spool 512) for another actuation stroke.

If desired, a user may actuate the lever 520 several times to advance the outer sheath 506 for compressing the basket 508, after which the outer sheath 506 preferably will have been advanced sufficiently to provide crushing force on the stone. The length of each stroke with the lever 520 may be selected by the user to advance the outer sheath 506 by a desired amount that may correspond to one or more of the ratchet teeth 506. It should be noted that the retaining pawl 536 or another gripping retaining member could be located distally of the driver pawl, or in another position. In alternative examples (not shown), the engagement of the pawls 530, 536 with the outer sheath 506 need not include ratchet teeth. For example, the surface of one or both pawls, the stem, and/or the sheath may be configured to engage frictionally.

In a preferred method of use, a user of the lithotriptor 500 first engages the basket 508 around the stone 511 and uses the handle 502 in a standard three-ring fashion to draw the basket 508 snugly around the stone 511 to crush it. If the force available by "thumb and fingers" actuation of the handle 502 in "traditional three-ring handle mode" is insufficient to crush the stone, the user then disengages his thumb from the thumb aperture 514 and braces the broad body 516 against his palm. The user also disengages his fingers from the finger apertures 518 in the spool 512 and uses them to actuate the lever one or more times in a compression stroke as described above with reference to FIG. 5B. The mechanical advantage provided by the lever 520 preferably moves the outer sheath 506 distally with greater force than is available in the "traditional three-ring handle mode" to compact the basket 508 and crush the stone 511. As described, the illustrated lithotriptor 500 provides for a first, direct mode of actuation and a second, assisted (i.e., providing mechanical advantage) mode of actuation without a need for providing or assembling additional tools. Of course, it should be noted that, for a small and/or weak stone 511, a user may elect to use only the first mode if it is sufficient to crush the stone. Likewise, it should also be noted that a user may elect to forgo the first mode of actuation and utilize only the second mode of actuation to crush the stone 511.

Those of skill in the art will appreciate that other examples of the devices and methods described above may be practiced within the scope of the present invention. For example, a lithotriptor device of the present invention may be used in a non-medical method such as, for example, to disrupt the integrity of an accretion (e.g., a crystalline mass, a blob of organic semisolids) in a mechanical device. It is therefore intended that the foregoing detailed description be regarded as illustrative rather than limiting. It should be understood that the following claims, including all equivalents, are intended to define the scope of this invention.

## Claims

1. A lithotriptor device comprising:
a proximal handle (402);
an elongate sheath (406) with a lumen extending longitudinally therethrough;
a basket wire(404); and
a basket (408) attached to the basket wire near a distal end thereof, wherein the wire extends through the lumen of the elongate sheath;
the handle comprising a first handle member (410) and a second handle member (412) axially movable along the first handle member, one of the first handle member and the second handle member connected to the elongate sheath, the other of the first handle member and the second handle member connected to the basket wire;
the first and second handle members each comprising an engagement member configured to be engaged by a hand of the user;
**characterised by** the second handle member comprising two lever (420,421) and pawl (430,431) assemblies, configured for operation in a scissors-like manner, wherein each pawl is rotatably attached near a first end (426,427) of the lever and is biased in engagement with an engagement portion (432) of the first handle member, and wherein a fulcrum (422) of the levers connects the levers to the second handle member.

2. The lithotriptor device of claim 1, wherein engagement portion of the first handle member comprises at least one ratchet-toothed surface that is engageable by the pawls.

3. The lithotriptor device of claim 1, wherein the second end of the levers comprises a loop structure configured for gripping.

4. The lithotriptor device of claim 1, wherein the handle has a first mode of operation comprising a direct axial sliding movement of the first handle member along the second handle member, and a second mode of operation providing a mechanical advantage greater than that of the first mode of operation; and
wherein the second mode of operation comprises providing a generally proximal movement of a second end of the levers, which forces the pawl against the engagement portion of the first handle member with sufficient force to move the elongate sheath relative to the wire.

5. The lithotriptor device of claim 1, wherein the first handle member comprises an attachment to the sheath; and the second handle member comprises an attachment to the wire;
the handle members and attachments are configured such that, when the second handle member is moved in a proximal direction relative to the first handle member, the wire is pulled in the proximal direction relative to the lumen of the sheath;
the levers being configured to provide mechanical advantage for moving the second handle member proximally relative to the first handle member.

6. The lithotriptor device of claim 5, wherein, when the second handle member is in a first position relative to the first handle member, the second handle member is separated from a proximal device end by a first distance; and
the basket and an adjacent portion of the wire extend beyond a distal end of the elongate sheath; and
wherein, when the second handle member is in a second position relative to the first handle member, the second handle member is separated from the proximal end by a second distance that is less than the first distance;
the basket and an adjacent portion of the wire are at least partially encompassed by the distal end of the elongate sheath; and
wherein an actuation of the lever moves the second handle member from the first position to the second position.

7. The lithotriptor device of claim 5, wherein each lever comprises a first lever end attached to the pawl, a second lever end opposite the first lever end, said fulcrum being disposed therebetween;
wherein the fulcrum is spaced closer to the first lever end than the second lever end such that a movement of the second lever end causes a relatively smaller movement of the first lever end.

## Patentansprüche

1. Lithotriptorvorrichtung, aufweisend:
einen proximalen Griff (402);
eine längliche Hülle (406) mit einem Lumen, das sich in Längsrichtung durch diese erstreckt;
einen Korbdraht (404); und
einen Korb (408), der am Korbdraht nahe seinem distalen Ende befestigt ist, wobei sich der Draht durch das Lumen der länglichen Hülle erstreckt;
wobei der Griff ein erstes Griffelement (410) und ein zweites Griffelement (412), das axial entlang des ersten Griffelements beweglich ist, aufweist, wobei eines von dem ersten Griffelement und dem zweiten Griffelement mit der länglichen Hülle verbunden ist, das andere von dem ersten Griffelement und dem zweiten Griffelement mit dem Korbdraht verbunden ist;
wobei das erste und zweite Griffelement jeweils ein Eingriffselement aufweisen, das dazu gestaltet ist, von einer Hand des Benutzers ergriffen zu werden;
**dadurch gekennzeichnet, dass** das zweite Griffelement zwei Hebelgruppen (420, 421) und Klinkengruppen (430, 431) aufweist, die für einen scherenartigen Betrieb gestaltet sind, wobei jede Klinke drehbar nahe einem ersten Ende (426, 427) des Hebels angeordnet ist und in Eingriff mit einem Eingriffsabschnitt (432) des ersten Griffelements vorgespannt ist, und wobei ein Drehpunkt (422) der Hebel die Hebel mit dem zweiten Griffelement verbindet.

2. Lithotriptorvorrichtung nach Anspruch 1, wobei ein Eingriffsabschnitt des ersten Griffelements zumindest eine gezahnte Oberfläche aufweist, die mit den Klinken in Eingriff gelangen kann.

3. Lithotriptorvorrichtung nach Anspruch 1, wobei das zweite Ende der Hebel eine Schlingenstruktur aufweist, die zum Ergreifen gestaltet ist.

4. Lithotriptorvorrichtung nach Anspruch 1, wobei der Griff einen ersten Betriebsmodus hat, der eine direkte axiale Gleitbewegung des ersten Griffelements entlang des zweiten Griffelements aufweist, und einen zweiten Betriebsmodus, der einen mechanischen Vorteil größer als jenen des ersten Betriebsmodus bietet; und
wobei der zweite Betriebsmodus ein Bereitstellen einer im Allgemeinen proximalen Bewegung eines zweiten Endes der Hebel aufweist, die die Klinke mit ausreichender Kraft gegen den Eingriffsabschnitt des ersten Griffelements presst, um die längliche Hülle relativ zum Draht zu bewegen.

5. Lithotriptorvorrichtung nach Anspruch 1, wobei das erste Griffelement eine Befestigung an der Hülle aufweist; und
das zweite Griffelement eine Befestigung am Draht aufweist;
wobei die Griffelemente und Befestigungen so gestaltet sind, dass, wenn das zweite Griffelement in eine proximale Richtung relativ zum ersten Griffelement bewegt wird, der Draht in die proximale Richtung relativ zum Lumen der Hülle bewegt wird;
wobei die Hebel zum Bereitstellen eines mechanischen Vorteils zum Bewegen des zweiten Griffelements proximal relativ zum ersten Griffelement gestaltet sind.

6. Lithotriptorvorrichtung nach Anspruch 5, wobei, wenn das zweite Griffelement in einer ersten Position relativ zum ersten Griffelement ist, das zweite Griffelement von einem proximalen Vorrichtungsende durch eine erste Distanz getrennt ist; und
der Korb und ein benachbarter Abschnitt des Drahtes sich über ein distales Endes der länglichen Hülle hinaus erstrecken; und
wobei, wenn das zweite Griffelement in einer zweiten Position relativ zum ersten Griffelement ist, das zweite Griffelement vom proximalen Ende durch eine zweite Distanz getrennt ist, die geringer als die erste Distanz ist;
der Korb und ein benachbarter Abschnitt des Drahtes zumindest teilweise vom distalen Ende der länglichen Hülle umschlossen sind; und
wobei die Betätigung des Hebels das zweite Griffelement aus der ersten Position in die zweite Position bewegt.

7. Lithotriptorvorrichtung nach Anspruch 5, wobei jeder Hebel ein erstes Hebelende, das an der Klinke angebracht ist, und ein zweites Hebelende gegenüber dem ersten Hebelende aufweist, wobei der Drehpunkt dazwischen angeordnet ist;
wobei der Drehpunkt näher zum ersten Hebelende als dem zweiten Hebelende beabstandet ist, sodass eine Bewegung des zweiten Hebelendes eine verhältnismäßig geringere Bewegung des ersten Hebelendes bewirkt.

## Revendications

1. Dispositif lithotriteur comportant :
une poignée (402) proximale ;
une gaine (406) allongée présentant une lumière qui s'étend longitudinalement à travers cette dernière ;
un fil métallique (404) de panier ; et
un panier (408) fixé au fil métallique de panier près d'une extrémité distale de ce dernier, dans lequel le fil métallique s'étend à travers la lumière de la gaine allongée ;
la poignée comportant un premier élément (410) poignée et un second élément (412) poignée mobile axialement le long du premier élément poignée, l'un des premier élément poignée et second élément poignée étant relié à la gaine allongée, l'autre des premier et second élément poignée étant relié au fil métallique de panier ;
les premier et second éléments poignée comportant chacun un élément de mise en prise configuré pour être mis en prise par la main d'un utilisateur ;
**caractérisé en ce que** le second élément poignée comporte deux ensembles levier (420, 421) et cliquet (430, 431), configurés pour fonctionner de la même manière que des ciseaux, dans lequel chaque cliquet est fixé de manière rotative près d'une première extrémité (426, 427) du levier et est sollicité afin de venir en prise avec une partie (432) de mise en prise du premier élément poignée, et dans lequel un pivot (422) des leviers relie les leviers au second élément poignée.

2. Dispositif lithotriteur selon la revendication 1, dans lequel la partie de mise en prise du premier élément poignée comporte au moins une surface de dent de rochet pouvant être mise en prise par les cliquets.

3. Dispositif lithotriteur selon la revendication 1, dans lequel la seconde extrémité des leviers comporte une structure en boucle configurée pour la saisie.

4. Dispositif lithotriteur selon la revendication 1, dans lequel la poignée a un premier mode de fonctionnement comportant un déplacement axial direct par coulissement du premier élément poignée le long du second élément poignée, et un second mode de fonctionnement fournissant un avantage mécanique plus grand que celui du premier mode de fonctionnement ; et
dans lequel le second mode de fonctionnement comporte la fourniture d'un déplacement généralement proximal d'une seconde extrémité des leviers, ce qui force le cliquet contre la partie de mise en prise du premier élément poignée avec une force suffisante pour déplacer la gaine allongée par rapport au fil métallique.

5. Dispositif lithotriteur selon la revendication 1, dans lequel le premier élément poignée comporte une fixation sur la gaine et le second élément poignée comporte une fixation sur le fil métallique ;
les éléments poignée et les fixations sont configurés de telle sorte que, lorsque que le second élément poignée est déplacé dans une direction proximale par rapport au premier élément poignée, le fil métallique est tiré dans la direction proximale par rapport à la lumière de la gaine ;
les leviers étant configurés pour fournir un avantage mécanique afin de déplacer le second élément poignée proximalement par rapport au premier élément poignée.

6. Dispositif lithotriteur selon la revendication 5, dans lequel, lorsque le second élément poignée est dans une première position par rapport au premier élément poignée, le second élément poignée est séparé d'une extrémité du dispositif proximal d'une première distance ; et
le panier et une partie adjacente du fil métallique s'étendent au-delà d'une extrémité distale de la gaine allongée ; et
dans lequel, lorsque le second élément poignée est dans une seconde position par rapport au premier élément poignée, le second élément poignée est séparé de l'extrémité proximale d'une seconde distance qui est inférieure à la première distance ;
le panier et une partie adjacente du fil métallique sont au moins partiellement contenus par l'extrémité distale de la gaine allongée ; et
dans lequel un actionnement du levier déplace le second élément poignée de la première position vers la seconde position.

7. Dispositif lithotriteur selon la revendication 5, dans lequel chaque levier comporte une première extrémité de levier fixée au cliquet, une seconde extrémité du levier opposée à la première extrémité de levier, ledit pivot étant disposé entre les deux ;
dans lequel le pivot est situé plus près de la première extrémité de levier que de la seconde extrémité de levier de telle sorte qu'un déplacement du second levier entraîne un déplacement relativement plus petit de la première extrémité de levier.
